# EUROPÄISCHE PATENTANMELDUNG

(11) **EP 4 847 484 A1**
(43) Veröffentlichungstag der Anmeldung: **02.12.2026**
(21) Anmeldenummer: 25179083.8
(22) Anmeldetag: 27.05.2025
(51) Int. Cl.: C07C 69/75, C08K 5/00, C08K 5/12

(54) **DIPENTYLESTER DER 1,4-CYCLOHEXANDICARBONSÄURE UND DIESE ENTHALTENDE WEICHMACHERZUSAMMENSETZUNGEN**

(71) Anmelder: Evonik Oxeno GmbH & Co. KG, 45772 Marl (DE)
(72) Erfinder: GRASS, Michael, 45721 Haltern am See (DE); VAN EICKELS, Michael, 45657 Recklinghausen (DE); RUSEK, Monika, 45149 Essen (DE); SCHAFFER, Timo, 45721 Haltern am See (DE); BECKER, Stephan, 45770 Marl (DE); BLEX, Christine, 45770 Marl (DE); FIEHN, Sophia, 46514 Schermbeck (DE)
(74) Vertreter: Evonik Patent Association

(57) **Zusammenfassung**

Die vorliegende Erfindung betrifft Dipentylester der 1,4-Cyclohexandicarbonsäure nach der Formel (1) 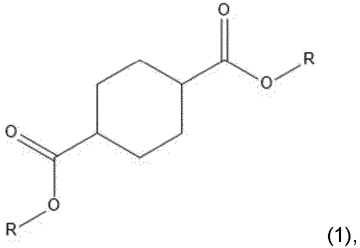 bei denen die beiden Alkylgruppen R jeweils 5 Kohlenstoffatome aufweisen, von denen 30 bis 97 Mol% n-Pentylreste sind. Ein weiterer Gegenstand ist eine Weichmacherzusammensetzung, die die vorgenannten Dipentylester der 1,4-Cyclohexandicarbonsäure nach der Formel (1) umfasst.

## Beschreibung

Gegenstand der Erfindung sind Dipentylester der 1,4-Cyclohexandicarbonsäure gemäß der allgemeinen Formel (1) 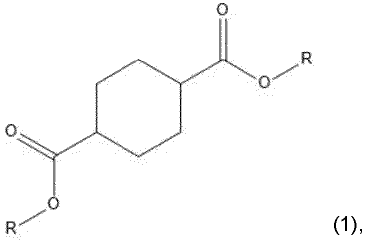 bei denen die beiden Alkylgruppen R jeweils 5 Kohlenstoffatome aufweisen, von denen 30 bis 97 Mol% n-Pentylreste sind. Ein weiterer Gegenstand ist eine Weichmacherzusammensetzung, die die vorgenannten Dipentylester der 1,4-Cyclohexandicarbonsäure nach der Formel (1) umfasst.

Die seitens der Verarbeiter von PVC-Plastisolen stetig steigenden Anforderungen hinsichtlich Verarbeitungsgeschwindigkeit sowie Möglichkeiten zur Energieeinsparung machen die Verwendung immer besser gelierender Weichmacher erforderlich. Diese Produkte, dem Fachmann als Schnellgelierer bekannt, können alleine oder auch in Kombination mit anderen Weichmachern eingesetzt werden.

Solche Kombinationen werden überwiegend in PVC-Plastisolen zur Herstellung von beispielsweise Fußbodenbelägen, Tapeten, Kunstledern oder auch Unterbodenschutzmassen eingesetzt.

Ein im Markt bekannter Schnellgelierer ist das Dipentylterephthalat, das großtechnisch unter dem Markennamen ELATUR^{®} DPT (kurz: DPT) von der Firma Evonik Oxeno GmbH & Co. KG erhältlich und u.a. in der EP3 351 526 A1 beschrieben ist.

Der EP 3 351 526 A1 ist allerdings zu entnehmen, dass man bei der Zusammensetzung der zur Herstellung der Ester eingesetzten linearen und verzweigten Pentanole, in der Regel n-Pentanol und 2-Methylbutanol, bezüglich des Anteils der n-Pentylreste im resultierenden Ester auf einen bestimmten Bereich limitiert ist, da der resultierende Ester außerhalb dieses Bereichs bei tiefer Temperatur entweder fest wird oder einen starken Viskositätsanstieg bei tiefen Temperaturen zeigt, was eine deutlich verringerte Pumpbarkeit bedeutet. Somit muss also die zur Herstellung von DPT benötigte Alkoholmischung aufwändig destilliert werden, was Ausbeuteverluste mit sich bringt. Großtechnische Prozesse zur Hydroformylierung von zum Beispiel n-Butenen liefern in der Regel einen Anteil von mindestens 80 Mol-%, eher > 90 Mol-% n-Pentanol nach Hydrierung der durch die Hydroformylierung erhaltenen Aldehyde. Reines n-Pentanol ist somit auch nicht ohne weitere destillative Trennung erhältlich, da es in der Regel immer zusammen mit 2-Methylbutanol gebildet wird.

Es wäre daher wünschenswert, aus den Alkoholgemischen, die direkt durch Hydroformylierung von n-Butenen und nachfolgender Hydrierung der erhaltenen Aldehyde, d.h. ohne weitere destillative Auftrennung der Alkohole zugänglich sind, einen Weichmacher produzieren zu können, der bei tieferen Temperaturen noch gut pumpbar ist und vor allem nicht zur Kristallisation neigt bzw. einen möglichst niedrigen Schmelzpunkt hat. Dies ist notwendig, da Lagertanks meist im Freien stehen und in der Regel beheizt werden müssen, um das zu verhindern. Ein Weichmacher mit den gewünschten Eigenschaften bei tieferer Temperatur könnte somit wegen des möglichen Verzichts auf eine Beheizung von Tank und Rohrleitungen dazu beitragen, Energie einzusparen.

Der vorliegenden Erfindung lag nun die Aufgabe zugrunde, eine Verbindung bereitzustellen, die gegenüber dem Dipentylterephthalat verbesserte Eigenschaften aufweist und bevorzugt über eine sehr ähnliche Rohstoffbasis zugänglich ist.

Diese Aufgabe wird durch die speziellen Dipentylester der 1,4-Cyclohexandicarbonsäure nach Anspruch 1 gelöst. Die Aufgabe wird weiterhin gelöst durch die erfindungsgemäße Weichmacherzusammensetzungen, die Dipentylester der 1,4-Cyclohexandicarbonsäure enthalten. Bevorzugte Ausführungsformen sind in den abhängigen Ansprüchen angegeben.

Ein Gegenstand der vorliegenden Erfindung sind Dipentylester der 1,4-Cyclohexandicarbonsäure nach der Formel (1) 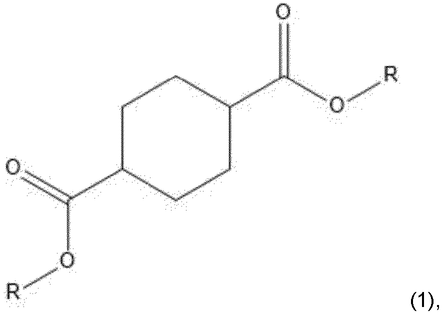 bei denen die beiden Alkylgruppen R jeweils 5 Kohlenstoffatome aufweisen, von denen 30 bis 97 Mol% n-Pentylreste sind. In einer bevorzugten Ausführungsform sind 55 bis 97 Mol% der Alkylgruppen R n-Pentylreste. In einer besonders bevorzugten Ausführungsform sind 60 bis 97 Mol% der Alkylgruppen R n-Pentylreste. In einer ganz besonders bevorzugten Ausführungsform sind 80 bis 97 Mol% der Alkylgruppen R n-Pentylreste. Die Prozentangabe Mol% bezieht sich auch hier jeweils auf 100 Mol% der Reste R, also alle Reste R der Verbindung nach Formel (1).

Im Rahmen der vorliegenden Erfindung werden die Begriffe "pentyl", "Pentylreste" oder ähnliches verwendet. Damit wird unterstrichen, dass die erfindungsgemäßen Ester verschiedene Pentyl-Isomere, insbesondere 2-Methylbutyl und/oder 3-Methylbutyl und/oder n-Pentyl, enthalten.

Die Reste R sind folglich Pentylreste und die erfindungsgemäßen Verbindungen nach Formel (1) sind demnach Dipentylester der 1,4-Cyclohexandicarbonsäure, die einen gewissen Anteil an einem oder mehreren Isomeren der Pentylgruppe enthalten. Wichtigste Vertreter bei den Isomeren der Pentylgruppe sind die lineare n-Pentylgruppe und die einfach verzweigten 2-Methylbutylgruppe und 3-Methylbutylgruppe.

Es ist erfindungsgemäß bevorzugt, dass der Dipentylester der 1,4-Cyclohexandicarbonsäure neben den n-Pentylresten ausschließlich 2-Methylbutyl- und/oder 3-Methylbutylreste umfasst. Die Summe aller vorhandenen Pentylreste im Mischester ergibt naturgemäß 100 Mol%. Somit sind 3 bis 70 Mol%, vorzugsweise 3 bis 45 Mol%, besonders bevorzugt 3 bis 40 Mol% und ganz besonders bevorzugt 3 bis 20 Mol% der Alkylgruppen R in den Dipentylestern der 1,4-Cyclohexandicarbonsäure nach der Formel (1) 2-Methylbutylreste und/oder 3-Methylbutylreste. Die Prozentangabe Mol% bezieht sich auch hier jeweils auf 100 Mol% der Reste R, also alle Reste R der Verbindung nach Formel (1).

Besonders bevorzugt enthalten die Pentylgruppen der Dipentylester der 1,4-Cyclohexandicarbonsäure nach der Formel (1) neben den n-Pentylresten nur 2-Methylbutylreste. Somit sind 3 bis 70 Mol%, vorzugsweise 3 bis 45 Mol%, vorzugsweise 3 bis 40 Mol% und ganz besonders bevorzugt 3 bis 20 Mol% der Alkylgruppen R in den Dipentylestern der 1,4-Cyclohexandicarbonsäure nach der Formel (1) 2-Methylbutylreste. Die Prozentangabe Mol% bezieht sich auch hier jeweils auf 100 Mol% der Reste R, also alle Reste R der Verbindung nach Formel (1).

Die beiden Pentylestergruppen der erfindungsgemäßen 1,4-Cyclohexandicarbonsäureester können sich auf unterschiedlichen oder gleichen Seiten der Molekülebene befinden (cis/trans-Isomerie). Es ist erfindungsgemäß bevorzugt, wenn die Dipentylester der 1,4-Cyclohexandicarbonsäure nach der Formel (1) einen cis-Gehalt von 60 bis 90 %, vorzugsweise 70 bis 85 % aufweisen. Der cis-Gehalt kann recht einfach mittels ¹H-NMR-Spektroskopie oder Gaschromatographie bestimmt werden. Es sei hierbei erwähnt, dass sich die Summe aus cis- und trans-Gehalt jeweils zu 100 % ergibt.

Ein weiterer Gegenstand der vorliegenden Erfindung ist eine Weichmacherzusammensetzung, die die vorher beschriebenen Dipentylester der 1,4-Cyclohexandicarbonsäure enthält. Das entspricht einer Weichmacherzusammensetzung, enthaltend Dipentylester der 1,4-Cyclohexandicarbonsäure nach der Formel (1) 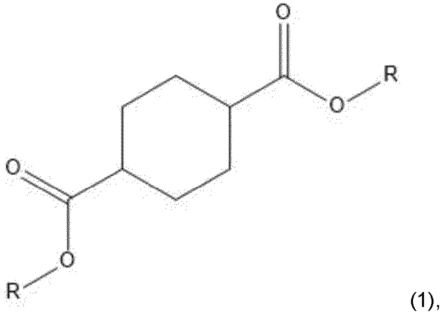 bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die Prozentangabe Mol% bezieht sich auch hier jeweils auf 100 Mol% der Reste R, also alle Reste R der Verbindung nach Formel (1).

Es ist erfindungsgemäß bevorzugt, dass der Dipentylester der 1,4-Cyclohexandicarbonsäure in der Weichmacherzusammensetzung neben den n-Pentylresten ausschließlich 2-Methylbutyl- und/oder 3-Methylbutylreste umfasst. Somit sind 3 bis 70 Mol%, vorzugsweise 3 bis 45 Mol%, besonders bevorzugt 3 bis 40 Mol% und ganz besonders bevorzugt 3 bis 20 Mol% der Alkylgruppen R in den Dipentylestern der 1,4-Cyclohexandicarbonsäure nach der Formel (1) 2-Methylbutylreste und/oder 3-Methylbutylreste.

Besonders bevorzugt enthalten die Pentylgruppen der Dipentylester der 1,4-Cyclohexandicarbonsäure in der Weichmacherzusammensetzung nach der Formel (1) neben den n-Pentylresten nur 2-Methylbutylreste. Somit sind 3 bis 70 Mol%, vorzugsweise 3 bis 45 Mol%, vorzugsweise 3 bis 40 Mol% und ganz besonders bevorzugt 3 bis 20 Mol% der Alkylgruppen R in den der Dipentylestern der 1,4-Cyclohexandicarbonsäure nach der Formel (1) 2-Methylbutylreste.

Die beiden Pentylestergruppen der erfindungsgemäßen 1,4-Cyclohexandicarbonsäureester können sich auf unterschiedlichen oder gleichen Seiten der Molekülebene befinden (cis/trans-Isomerie). Es ist erfindungsgemäß bevorzugt, wenn die Dipentylester der 1,4-Cyclohexandicarbonsäure nach der Formel (1) einen cis-Gehalt von 60 bis 90 %, vorzugsweise 70 bis 85 % aufweisen. Der cis-Gehalt kann recht einfach mittels ¹H-NMR-Spektroskopie oder Gaschromatographie bestimmt werden. Es sei hierbei erwähnt, dass sich die Summe aus cis- und trans-Gehalt jeweils zu 100 % ergibt.

Eine systematische Untersuchung der Tieftemperatureigenschaften der Dipentylester der 1,4-Cyclohexandicarbonsäure zeigt, dass die gewünschte Verarbeitbarkeit bei tiefen Temperaturen bis zu -10 °C gegeben ist. Weiterhin zeigen diese erfindungsgemäßen Ester auch gute Eigenschaften als schnell gelierender Weichmacher.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Isosorbidestern, Furandicarboxylaten, Phosphaten, Succinaten, Sulfonamiden, Alkylsulfonsäureestern des Phenols, Terephthalaten, Isophthalaten, Phthalaten, Trimellitaten, Cyclohexantripropionsäuretrialkylestern, Cyclohexandicarboxylaten und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6-Alkansäureester von Polyolen, acetylierten oder nicht acetylierten Citronensäuretrialkylestern, Glykoldibenzoaten, Trialkylestern der Trimellitsäure, 1,2,4-Cyclohexantripropionsäuretrialkylestern, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate und Dialkylestern der 1,2-, 1,3- oder 1,4-Cyclohexandicarbonsäure, ausgewählt wird.

Die beiden Substanzen, d. h. die Dipentylester der 1,4-Cyclohexandicarbonsäure und der weitere Weichmacher, können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen die Verbindung nach Formel (1) und weitere Weichmacher in einem Gewichtsverhältnis (Verbindung nach Formel (1) : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern und je nach ihrem Anteil auch weichmachende Eigenschaften aufweisen.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden.

Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus der Verbindung gemäß Formel (1) und dem weiteren Weichmacher eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Gesamtsumme aus der Verbindung gemäß Formel (1) und dem weiteren Weichmacher in der Weichmacherzusammensetzung enthalten sein.

In einer weiterhin bevorzugten Ausführungsform wird der mindestens ein weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, C4-bis C10-Trialkyltrimellitaten, C4- bis C9-Dialkylterephthalaten, C4-C10-Isophthalaten, C4- bis C13-Dialkylphthalaten, insbesondere C9- bis C13-Dialkylphthalaten, C1-C10-Trialkylester der 1,2,4-Cyclohexantripropionsäure und C4- bis C10-Dialkylestern der 1,2-, 1,3- oder 1,4-Cyclohexandicarbonsäure, ausgewählt.

Die Auswahl einer geeigneten Kombination von Weichmachern zur Mischung mit den erfindungsgemäßen Dipentylestern der 1,4-Cyclohexandicarbonsäure nach der oben gezeigten Formel (1) ist für den Fachmann grundsätzlich Routine. Die Auswahl einer Weichmacherkombination richtet sich immer nach der betreffenden Anwendung und den kommerziell erhältlichen Weichmachern sowie deren Eigenschaften. Nachfolgend sind besonders bevorzugte Weichmacherkombination aufgelistet.

Ein Gegenstand der vorliegenden Erfindung ist eine Weichmacherzusammensetzung, umfassend Tributylcitrat und die Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tripentylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-n-hexylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-2-ethylhexylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triisononylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltributylcitrat (ATBC) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltripentylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltri-2-ethylhexylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Acetyltri-isononylcitrat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diethylhexyladipat (DEHA) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diisononyladipat (DINA) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diethylhexylterephthalat (DEHT bzw. DOTP) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triethylhexyltrimellitat (TEHTM bzw. TOTM) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Triisononyltrimellitat (TINTM) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tripentyltrimellitat (TPTM) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tributyltrimellitat (TBTM) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,4-Diethylhexylcyclohexandicarbonsäureester (1,4-DEHCH) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,2-Diethylhexylcyclohexandicarbonsäureester (1,2-DEHCH) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,4-Diisononylcyclohexandicarbonsäureester (1,4-DINCH oder DINCD) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend den 1,2-Diisononylcyclohexandicarbonsäureester (DINCH) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diethylhexylphthalat (DEHP oder DOP) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diisononylphthalat (DINP) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Dipropylheptylphthalat (DPHP) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Diisodecylphthalat (DIDP) und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Pentaerythrit-tetravalerat und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend ein Glycoldibenzoat aus der Gruppe, bestehend aus Diethylenglycoldibenzoat, Dipropylenglycoldibenzoat, Triethylenglycoldibenzoat, und Mischungen davon, und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tripentyl-Cyclohexan-1,2,4-tripropionsäureester und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Trimethyl-Cyclohexan-1,2,4-tripropionsäureester und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tributyl-Cyclohexan-1,2,4-tripropionsäureester und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-2-ethylhexyl-cyclohexan-1,2,4-tripropionsäureester und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Weichmacherzusammensetzung, umfassend Tri-isononyl-cyclohexan-1,2,4-tripropionsäureester und Dipentylester der 1,4-Cyclohexandicarbonsäure nach der oben gezeigten Formel (1), bei denen die beiden Alkylgruppen R jeweils 5 Kohlenstoffatome aufweisen, von denen 30 bis 97 Mol%, vorzugsweise 55 bis 97 Mol%, besonders bevorzugt 60 bis 97 Mol% und ganz besonders bevorzugt 80 bis 97 Mol% n-Pentylreste sind. Die beiden Weichmacher liegen vorzugsweise in einem Gewichtsverhältnis von 1 : 99 bis 99 : 1, vorzugsweise 10 : 90 bis 90 :10, weiterhin bevorzugt 30 : 70 bis 70 : 30 oder 50 : 50 vor.

Ein weiterer Gegenstand der vorliegenden Erfindung ist eine Kunststoffzusammensetzung, die einen Kunststoff und die Weichmacherzusammensetzung, umfassend die Verbindung der Formel (1) und optional einen weiteren Weichmacher enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff ein Polymer ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und CoPolymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel, UV-Stabilisatoren und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1 - Herstellung von Dipentylestern der 1,4-Cyclohexandicarbonsäure

Zunächst wurden die entsprechenden Mischungen aus n-Pentanol (Pe), sowie 2-Methylbutanol (Mb) und Cyclohexan-1,4-dicarbonsäure (siehe Tabelle 1) inklusive eines 25% molaren Alkoholüberschusses in Bezug auf die Menge an eingesetzter Cyclohexan-1,4-dicarbonsäure in einer Veresterungsapparatur bestehend aus Dreihalskolben, Innenthermometer, Wasserauskreiser mit aufgesetztem Rückflusskühler, Rührwerk und einem Tauchrohr vorgelegt. Vor der Zugabe des Katalysators Tetra(n-butyl)titanat (TNBT) (0,1 mol % bezogen auf Cyclohexan-1,4-dicarbonsäure) wurde die gesamte Apparatur mit Stickstoff gespült. Danach wurde die Reaktion durch Aufheizen gestartet. Die Reaktionstemperatur betrug dabei 130 - 240°C, d. h. sie stieg während der Reaktion an. Entstehendes Reaktionswasser wurde über den Wasserauskreiser aus dem System entfernt, sodass das Gleichgewicht in Richtung Vollumsatz verschoben werden konnte.

Sobald kein Wasseranfall mehr zu beobachten war, wurde eine Probe gezogen und die Säurezahl bestimmt. Bei einer Säurezahl < 0,5 mg KOH pro g Probe, wurde die Reaktion beendet, indem die Heizung abgestellt wurde.

Im nächsten Schritt wurde der überschüssige Alkohol bei bis zu 160 °C unter Vakuum abdestilliert. Anschließend wurde die Säurezahl der Reaktionsmischung bestimmt und diese dann durch die Zugabe der 3-fachen stöchiometrischen Menge an 10%-NaOH neutralisiert. Restmengen an Alkohol wurden im nächsten Schritt durch eine Wasserdampfdestillation bei 160 °C unter Vakuum entfernt. Anschließend wurde der Ansatz bei 130°C unter Vakuum getrocknet, bis der Wassergehalt < 0,05 % war. Im letzten Schritt wurde das Produkt über einen 0,5 mm PTFE-Filter und Perlite als Filterhilfsmittel klar filtriert und mittels GC analysiert.

Der cis-Gehalt der Produkte wurden mittels Gaschromatographie bestimmt. Die Bestimmung wurde mit einem Agilent 7890 GC mit einer Agilent DB5 Säule und einem FID-Detektor durchgeführt. Vor der Injektion wird die Probe 1 zu 10 mit Aceton verdünnt. Nach der Injektion wird die Temperatur von 50 °C Injektionstemperatur auf 350 °C Endtemperatur mit einer Rampe von 7,5°C/min erhöht. Die Endtemperatur von 350°C wird für 10 Minuten gehalten. Als Carriergas wird Helium genutzt und als Make-Up Gas Stickstoff. Die Bestimmung des cis-Gehalts geschieht durch die Integration der Peakflächen der jeweiligen cis- und trans-Isomere und der Berechnung des cis-Gehalts basierend auf diesen Peakflächen. Der entsprechende trans-Gehalt ergibt sich dann durch Subtraktion des cis-Anteils von 100%.

**Tabelle 1: Pentanolmischungen für die Herstellung der Dipentylester der 1,4-Cyclohexandicarbonsäure**

| | Cyclohexan-1,4-dicarbonsäure / Gramm (mol) | Menge n-Pentanol (Pe) / Gramm (mol) | Menge 2-Methylbutanol (Mb) / Gramm (mol) | cis-Gehalt in % |
|---|---|---|---|---|
| 1a (Mb) | 550 (3,2) | 0 (0) | 705 (8,0) | 74 |
| 1b* (Pe/Mb = 50/50) | 585 (3,4) | 374 (4,3) | 374 (4,3) | 74 |
| 1c* (Pe/Mb = 65/35) | 1377 (8,0) | 1145 (13,0) | 617 (7,0) | 75 |
| 1d* (Pe/Mb = 80/20) | 1464 (8,5) | 1499 (17,0) | 374 (4,3) | 75 |
| 1e* (Pe/Mb = 93/7) | 1101 (6,4) | 1311 (14,8) | 99 (1,1) | 75 |
| 1f (Pe) | 576 (3,3) | 745 (8,5) | 0 | 73 |

| | | | | |
|---|---|---|---|---|
| * erfindungsgemäß | | | | |

### Beispiel 2 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezepturen der Polymerzusammensetzung sind in Tabelle 2 aufgelistet.

**Tabelle 2: Übersicht über die hergestellten Plastisole**

| Plastisol | A | B* | C* | D* | E* | F | G |
|---|---|---|---|---|---|---|---|
| | phr | phr | phr | phr | phr | phr | phr |
| PVC (Vestolit^{®} P 1430 K 70 Ultra; Fa. Vestolit) | 100 | 100 | 100 | 100 | 100 | 100 | 100 |
| Ester gemäß Beispiel 1a | 50 | | | | | | |
| Ester gemäß Beispiel 1b* | | 50 | | | | | |
| Ester gemäß Beispiel 1c* | | | 50 | | | | |
| Ester gemäß Beispiel 1d* | | | | 50 | | | |
| Ester gemäß Beispiel 1e* | | | | | 50 | | |
| Ester gemäß Beispiel 1f | | | | | | 50 | |
| ELATUR^{®} DPT (Dipentylterephthalat), Fa. Evonik Oxeno GmbH & Co. KG | | | | | | | 50 |
| epoxidiertes Sojabohnenöl als Co-Stabilisator (Edenol^{®} D81, Fa. Emery) | 3 | 3 | 3 | 3 | 3 | 3 | 3 |
| Thermostabilisator auf Ca/Zn-Basis (Reagens MBL 197/9 PF, Fa. Reagens) | 2 | 2 | 2 | 2 | 2 | 2 | 2 |

| | | | | | | | |
|---|---|---|---|---|---|---|---|
| * = erfindungsgemäß phr = parts per hundred parts resin | | | | | | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 3 - Viskositäten der Plastisole

Die Messung der Viskosität der in Beispiel 3 hergestellten Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden.

Während der Messung wurden folgende Punkte angesteuert:
- Vorscherung von 100 s⁻¹ für einen Zeitraum von 60 Sekunden, bei der keine Messwerte aufgenommen wurden
- Scherraten-Abwärtsrampe von 200 s⁻¹ bis 0,1 s⁻¹. Es wurden 30 Messpunkte aufgenommen mit einer Messpunktdauer von je 10 Sekunden.

Die Messungen wurden bei Raumtemperatur durchgeführt. Die Ergebnisse der Viskositätsmessungen der in Beispiel 2 hergestellten Plastisole mit den jeweils angegebenen Weichmachern sind in Tabelle 3 gezeigt.

**Tabelle 3: Viskosität der Plastisole in Pa*s nach 2 h bei einer Schergeschwindigkeit von 200 s⁻¹**

| Rezeptur | Anteil n-Pentanol/2-Methylbutyl in Mol-% | Viskosität nach 2 h / Pa*s |
|---|---|---|
| A | 0/100 | 2,991 |
| B* | 50/50 | 2,86 |
| C* | 65/35 | 2,518 |
| D* | 80/20 | 2,333 |
| E* | 93/7 | 2,66 |
| F | 100/0 | 2,592 |
| G | 50/50 | 4,730 |

| | | |
|---|---|---|
| * erfindungsgemäß | | |

Die erfindungsgemäßen Weichmacher B bis E weisen im Plastisol deutlich niedrigere Viskositäten auf als DPT (G).

### Beispiel 4 - Gelierung der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |
| Automatische Spaltnachführung F: | 0 N |
| Konstante Messpunktdauer Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 4 aufgeführt.

**Tabelle 4: Geliertemperatur in °C bei Erreichen einer Pastenviskosität von 1000 Pa*s.**

| Rezeptur | Anteil n-Pentanol/2-Methylbutyl in Mol-% | Geliertemperatur [°C] |
|---|---|---|
| A | 0/100 | 64,8 |
| B* | 50/50 | 63,1 |
| C* | 65/35 | 62,7 |
| D* | 80/20 | 62,5 |
| E* | 93/7 | 62,4 |
| F | 100/0 | 62,2 |
| G | 50/50 | 67,4 |

| | | |
|---|---|---|
| * erfindungsgemäß | | |

Durch einen höheren n-Pentyl-Anteil der erfindungsgemäßen Tripentylester kann die Geliertemperatur abgesenkt werden. Bei allen Estern gemäß Formel 1 ist diese geringer als beim DPT. Der Übergang vom Terephthalat DPT (G) zum jeweiligen 1,4-Cyclohexandicarbonsäureester mit gleicher Alkoholzusammensetzung (B) bringt eine Absenkung von mehr als 4°C.

### Beispiel 5 - Herstellung von Folien

Die im Beispiel 2 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 6 - Bestimmung der Glasübergangstemperatur mittels DMTA

Die Glasüberganstemperatur wurde mit Hilfe von DMTA-Messungen gemäß DIN 65583 mit einem Rheometer der Firma Anton Paar vom Typ MCR 302 bestimmt. Unter konstanten dynamischmechanischen Bedingungen (1 Hz, Deformation 0,3 %) wurden die viskoelastischen Eigenschaften der Folien (1 mm Dicke) in Abhängigkeit der Temperatur (Temperaturrampe von -100 bis +50 °C) erfasst und der Speichermodul, das Verlustmodul und der Verlustfaktor bestimmt. Das Maximum des Verlustmoduls wird dabei als Glasübergangstemperatur ausgewertet. Die nachfolgende Tabelle 5 zeigt jeweils den Mittelwert aus einer Doppelbestimmung.

**Tabelle 5: Glasübergangstemperaturen des Estergemische.**

| Foliennr. | Anteil n-Pentanol/2-Methylbutyl in Mol-% | Glasübergangstemperatur der Folien in °C |
|---|---|---|
| A | 0/100 | -31,8 |
| B* | 50/50 | -37,9 |
| C* | 65/35 | -40,0 |
| D* | 80/20 | -41,9 |
| E* | 93/7 | -42,9 |
| F | 100/0 | -43,8 |
| G | 50/50 | -29,2 |

| | | |
|---|---|---|
| * = erfindungsgemäß | | |

Die erfindungsgemäßen Weichmacher B bis E zeigen gegenüber DPT (G) eine teils deutlich verbesserte Glasübergangstemperatur.

### Beispiel 7 - Bestimmung der Shore A Härte

Aus den nach Beispiel 5 hergestellten Folien wurden 4,8 x 4,8 cm² große Stücke geschnitten. Diese wurden in einen Pressrahmen zu 6-8 Lagen, je nach Folienstärke, gestapelt und bei 170 °C und 200 bar verpresst. Hiernach wurden die Prüfkörper für mind. 16 h im Klimaraum unter Normklima (25°C, 50 % Raumfeuchte) konditioniert und anschließend in Anlehnung an DIN 48-4 mit einem Shore-A-Messgerät der Fa. Zwick-Roell vermessen. Dabei wurden die Messwerte nach 3 Sekunden abgelesen. An jedem Prüfkörper wurden Messungen an drei verschiedenen Stellen durchgeführt und der Mittelwert gebildet. Die Ergebnisse sind in Tabelle 6 aufgeführt.

**Tabelle 6: Shore A Härten der gepressten Prüfkörper.**

| Rezeptur | Anteil n-Pentanol / 2-Methylbutyl in Mol-% | Shore A |
|---|---|---|
| A | 0/100 | 75 |
| B* | 50/50 | 74 |
| C* | 65/35 | 74 |
| D* | 80/20 | 74 |
| E* | 93/7 | 73 |
| F | 100/0 | 73 |
| G | 50/50 | 76 |

| | | |
|---|---|---|
| * = erfindungsgemäße Zusammensetzung | | |

Durch einen höheren n-Pentyl-Anteil der erfindungsgemäßen Tripentylester kann die Shore A Härte abgesenkt und die Effizienz somit gesteigert werden. Gegenüber DPT (G) sind alle erfindungsgemäßen Weichmacher B bis E zumindest leicht verbessert.

### Beispiel 8 - Tieftemperaturviskosität der Estergemische

Mit diesem Versuch sollte ermittelt werden, ob beim Abkühlen der Ester eine plötzliche, starke Viskositätserhöhung eintritt, die anzeigt, dass die Mischung nicht mehr pumpbar wäre. Als Kriterium wurde festgelegt, die Temperatur zu bestimmen, bei der die Viskosität auf 1000 mPa*s ansteigt.

Die Viskositäten der Estergemische wurden an einem Rheometer MCR 302 (Fa. Anton Paar Germany GmbH) bestimmt. Das Rheometer ist wie folgt ausgestattet:

| | |
|---|---|
| Temperierofen: | CTD 450, Fa. Anton Paar Germany GmbH |
| Messsystem: | PP50 (Platte-Platte System), Fa. Anton Paar Germany GmbH |
| Stickstoffverdamper: | EVU 20, Fa. Anton Paar Germany GmbH |
| Gegentemperierthermostat: | 200F, Fa. Julabo GmbH |
| Stickstoffbehälter: | Apollo 100, Fa. Cryotherm GmbH & Co. KG |

Die Messung wurde bei 25 °C gestartet. Mittels einer Einwegpipette wurden die Probe auf die untere Platte aufgegeben. Danach wurde auf Trimmposition gefahren sowie hiernach auf einen Spaltabstand von 0,2 mm. Anschließend wurde die Temperiereinheit CTD 450 geschlossen und folgendes Messprogramm wurde gestartet:

### Abschnitt 1

78 Punkte, 0,167 min
γ = 0,1 %
ω = 10 rad/s
Temperatur: 25 bis -40 °C
F_{N} = 0 N

### Abschnitt 2

10 Punkte, 1 min
γ = 0,1 %
ω = 10 rad/s
Temperatur: -40 °C
F_{N} = 0 N

### Abschnitt 3

78 Punkte, 0,167 min
γ = 0,1 %
ω = 10 1/s
Temperatur: -40 bis 25 °C
F_{N} = 0 N

Die Auswertung der Viskositäten erfolgte anhand der Rheologie Software Rheoplus. Durch Interpolation der Messdaten der komplexen Viskosität wurde die Temperatur bei 1000 Pas bestimmt. In den meisten Fällen wurde dieser Wert von 1000 Pa*s auch bei der tiefsten Temperatur von - 40 °C nicht erreicht, die Produkte waren also ausreichend fließfähig. In Tabelle 7 sind die auf diese Weise ermittelten Temperaturen aufgeführt.

**Tabelle 7: Temperaturen bei Erreichen einer Viskosität von 1000 Pas mittels Interpolation.**

| | Anteil n-Pentanol / 2-Methylbutanol in Mol-% | Temperatur bei 1000 Pas [°C] |
|---|---|---|
| Ester gemäß Beispiel 1a | 0/100 | < -40 |
| Ester gemäß Beispiel 1b* | 50/50 | < -40 |
| Ester gemäß Beispiel 1c* | 65/35 | < -40 |
| Ester gemäß Beispiel 1d* | 80/20 | < -40 |
| Ester gemäß Beispiel 1e* | 93/7 | -11,13 |
| Ester gemäß Beispiel 1f | 100/0 | -7,03 |
| ELATUR^{®} DPT (Dipentylterephthalat) | 50/50 | < -40 |

Alle erfindungsgemäßen Weichmacher 1b* bis 1e* erfüllen die Voraussetzung, dass der starke Viskositätsanstieg erst bei Temperaturen < - 10 °C erfolgen sollte. Einzig der Weichmacher 1f erfüllt diese Voraussetzung nicht.

### Beispiel 9 - Erstarrungspunkt- und Trübungsverhalten der Estergemische

Jeweils ca. 20 g Weichmacher wurden in 20 mL Bördelrandflaschen gefüllt und verschlossen. In einen mit Ethanol befüllten Kryostaten (RL6, Fa. Lauda) wurden diese Fläschchen durch zwei beschwerende Probenhalter hereingestellt. Die Temperierung wurde bei 25 °C gestartet und um jeweils 5 °C / 24 h erniedrigt, bis eine Temperatur von -15 °C erreicht wurde. Nach den jeweiligen Temperierzeiten wurden die Flüssigkeiten in den Fläschchen hinsichtlich einer Kristallbildung beurteilt. Die zugehörigen Ergebnisse sind in Tabelle zusammengestellt.

**Tabelle 8: Ergebnisse der Erstarrungspunkt-Bestimmung.**

| Rezeptur | Anteil n-Pentanol/2-Methylbutyl in Mol-% | Feststoff vorhanden bei -10 °C? |
|---|---|---|
| Ester gemäß Beispiel 1a | 0/100 | nein |
| Ester gemäß Beispiel 1b* | 50/50 | nein |
| Ester gemäß Beispiel 1c* | 65/35 | nein |
| Ester gemäß Beispiel 1d* | 80/20 | nein |
| Ester gemäß Beispiel 1e* | 93/7 | nein |
| Ester gemäß Beispiel 1f | 100/0 | ja |
| ELATUR^{®} DPT (Dipentylterephthalat) | 50/50 | nein |

Die Ergebnisse in Beispiel 9 entsprechen somit den Ergebnissen aus dem Beispiel 8.

Insgesamt zeigt sich, dass das DPT (G) zwar bei tiefen Temperaturen auch noch fließfähig ist (Beispiele 8 und 9), allerdings in den anderen anwendungstechnischen Eigenschaften (Beispiele 3 bis 7) zum Teil deutlich schlechter performt als die erfindungsgemäßen Weichmacher 1b* bis 1e*. Der reine n-Pentylester (1f) weist hingegen keine guten Tieftemperatureigenschaften auf, wohingegen der reine 2-Methylbutylester (1a) schlechtere anwendungstechnische Eigenschaften aufweist.

## Patentansprüche

1. Dipentylester der 1,4-Cyclohexandicarbonsäure nach der Formel (1) 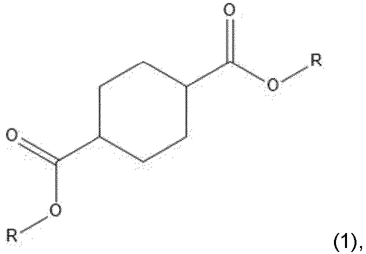 wobei die beiden Alkylgruppen R jeweils 5 Kohlenstoffatome aufweisen, von denen 30 bis 97 Mol% n-Pentylreste sind.

2. Dipentylester der 1,4-Cyclohexandicarbonsäure nach Anspruch 1, wobei 55 bis 97 Mol%, vorzugsweise 60 bis 97 Mol%, besonders bevorzugt 80 bis 97 Mol% der Alkylgruppen R n-Pentylreste sind.

3. Dipentylester der 1,4-Cyclohexandicarbonsäure nach Anspruch 1 oder 2, wobei 3 bis 70 Mol%, vorzugsweise 3 bis 45 Mol%, weiterhin bevorzugt 3 bis 40 Mol%, besonders bevorzugt 3 bis 20 Mol% der Alkylgruppen R 2-Methylbutylreste und/oder 3-Methylbutylreste sind.

4. Dipentylester der 1,4-Cyclohexandicarbonsäure nach Anspruch 3, wobei 3 bis 70 Mol%, vorzugsweise 3 bis 45 Mol%%, weiterhin bevorzugt 3 bis 40 Mol%, besonders bevorzugt 3 bis 20 Mol% der Alkylgruppen R 2-Methylbutylreste sind.

5. Weichmacherzusammensetzung, wobei die Weichmacherzusammensetzung die Dipentylester der 1,4-Cyclohexandicarbonsäure nach einem der Ansprüche 1 bis 4 enthält.

6. Weichmacherzusammensetzung nach Anspruch 5, wobei die Weichmacherzusammensetzung mindestens einen weiteren Weichmacher umfasst, der aus der Gruppe, bestehend aus Alkylbenzoaten, Alkylsulfonsäureestern des Phenols, Dialkyladipaten, Glycerinestern, C4- bis C6-Alkansäureester von Polyolen, acetylierten oder nicht acetylierten Citronensäuretrialkylestern, Glykoldibenzoaten, Trialkylestern der Trimellitsäure, 1,2,4-Cyclohexantripropionsäuretrialkylestern, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate und Dialkylestern der 1,2-, 1,3- oder 1,4-Cyclohexandicarbonsäure, ausgewählt wird.

7. Weichmacherzusammensetzung nach einem der vorhergehenden Ansprüche, wobei die Verbindung nach Formel (1) und der weitere Weichmacher (Verbindung nach Formel (1) : weiterer Weichmacher) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

8. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

9. Weichmacherzusammensetzung nach Anspruch 8, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

10. Weichmacherzusammensetzung nach Anspruch 9, wobei das epoxidierte Öl epoxidiertes Sojabohnenöl oder epoxidiertes Leinöl, vorzugsweise epoxidiertes Sojabohnenöl ist.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und CoPolymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 14 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.
